# EUROPEAN PATENT SPECIFICATION

(11) **EP 3 518 997 B1**
(45) Date of publication and mention of the grant of the patent: **28.10.2020**
(21) Application number: 17791461.1
(22) Date of filing: 29.09.2017
(51) Int. Cl.: A61L 27/18, A61L 27/24, A61L 27/58

(54) **PRODUCT FOR THE RECONSTRUCTION OF CARTILAGE**
PRODUKT ZUR REKONSTRUKTION VON KNORPELGEWEBE
PRODUIT POUR LA RECONSTRUCTION DE CARTILAGE

(30) Priority: 30.09.2016 IT 201600098385
(43) Date of publication of application: 07.08.2019
(73) Proprietor: Sambusseti, Antonio, 26100 Cremona (IT)
(72) Inventor: Sambusseti, Antonio, 26100 Cremona (IT)
(74) Representative: Torti, Carlo Maria Emilio
(86) International application number: PCT/IB2017/056000
(87) International publication number: WO 2018/060944

(56) References cited:
- US-A- 5 356 629
- US-A1- 2005 249 773
- US-A1- 2008 071 385

## Description

### Field of the invention

The present invention relates to a reabsorbable product for the reconstruction of cartilage and/or bone parts, for example a joint.

By way of example, the present invention is applicable to the reconstruction of cartilage and/or bone parts damaged by traumas or degenerative diseases of the joints, such as the knee, shoulder or others.

### Background art

According to the prior art, in the case of joint traumas or degenerative joint diseases, which cause a cartilage tissue and/or bone part injury, surgery is required to restore the situation and allow easy movement of the joint. In particular, during the surgery carried out by means of arthrotomy or arthroscopy, fragments of cartilage and damaged cartilage are removed from the joint area and/or bone parts are removed and replaced with prostheses.

After such operations, blood is taken from the patient and placed in a culture to obtain protein-rich plasma therefrom. A matrix is then implanted on the bone of the joint involved and irrigated with the culture.

This technique has several significant disadvantages. The cartilage cell culture is a particularly expensive process and takes a long time to be completed. Furthermore, this technique forces the patient to undergo various surgical treatments. In fact, the grafting of cultivated cells occurs after the surgery and further support therapies may be required, even during the tissue regeneration.

In this context, the technical task underlying the present invention is to propose a reabsorbable product for the reconstruction of cartilage and/or bone parts which overcomes the drawbacks of the above-mentioned prior art.

In particular, it is the object of the present invention to provide a reabsorbable product for the reconstruction of cartilage and/or bone parts which allows a rapid reconstruction of the cartilage and/or bone parts of a patient's joint and which allows a significant reduction in times and discomfort for the patient himself/herself. US5356629 A discloses a moldable composition for effecting bone repair comprising a plurality of biocompatible particles dispersed in a matrix, such as collagen. The particles can be biodegradable and derived from polyglycolic acid and they have an average size of 0.1 to 3 mm. Particles having an average size of 100 to 700 µm can also be utilized. The particles can be non-biodegradable, such as hydroxyapatite.

### Summary of the invention

The specified technical task and the object are substantially achieved by means of a reabsorbable product for the reconstruction of cartilage and/or bone parts comprising the technical features set out in one or more of the appended claims.

### Detailed description of embodiments

According to a first embodiment, the product according to the present invention relates to a device for regenerating cartilage and/or bone parts comprising PGA granules. In preferred embodiments of the invention, the product can then be fixed onto a patient's joint bone. However, the invention is not limited to this type of use and forms of use where the device is used for regenerating cartilage in vitro will be possible.

PGA, also known as polyglycolic acid or polyglycolide, is a highly biocompatible and reabsorbable polymer. In detail, the reabsorption time for PGA is about one month. In a specific embodiment of the invention, the device comprises PGA in granules having a dimension from 400 micrometres to 500 micrometres and a substantially spherical and/or round regular shape, where the best results in terms of regeneration of both cartilage and bone parts are obtained with PGA of the aforesaid dimensions and shapes and/or in powder form.

Nevertheless, satisfactory results can also be obtained with granules having dimensions from 100 to 900 micrometres, preferably from 200 to 700, even more preferably from 400 to 500.

Furthermore, embodiments of the product are possible in the scope of the present invention, according to which the PGA grains and/or powder are mixed with other substances and/or materials.

According to one embodiment, the granules having the desired dimensions are obtained by grinding, where the dimension thereof is thus constant and uniform after being set on the grinding machine.

The grinding operation does not allow serious errors to be made and even small size deviations are avoided, the deviations being not greater than 0.1% for 50% of granules, greater deviations affecting proportionately smaller percentages of granules.

According to a further embodiment, the PGA granules (and/or powder) are mixed with collagen to form a gelatinous mass, which is particularly advantageous because it is adapted, on the one hand, to be packaged in vials, and to be also spread and injected into the implant point and stay in place without other types of aids.

For example, in the scope of the present invention, collagen of porcine and/or bovine cartilage may be used.

Furthermore, in addition to the collagen, other additives mixed with PGA may be included, such as for example biological fibrin (as an amalgamative and adhesive, as well as an additional growth factor), in such a percentage as to allow a predetermined minimum percentage of PGA+collagen.

According to a further embodiment, which is particularly advantageous for the reconstruction of bone parts, the product is obtained by mixing:
- 1kg PGA corresponding to the standard ASTM F2313-08 Standard IV (dL/g): 1.10-1.20 Tg: 41 °C Tm: 223°C Max H2O: 0.05%;
- 0.5kg Hydroxylapatite powder, medical grade, with nominal dimension of 20 micrometres
with the possible addition of collagen, preferably purified, exceeding 95%, preferably of bio-absorbable I type.

According to the present invention, the product according to the fourth embodiment described above can be obtained according to a method comprising:
- cryogenic grinding 1 Kg of PGA, preferably in an ISO class 7 clean room, with a medical grinder set on the required dimension. For example, in the case of PGA having particles from 400 micrometres to 500 micrometres, the grinder would be set with the minimum and maximum values at 400 micrometres and 500 micrometres, respectively;
- possibly mixing the thus obtained PGA with hydroxylapatite, 70% by weight PGA and 30% by weight hydroxylapatite, preferably in an ISO class 8 clean room;
- mixing with dry collagen, preferably in an ISO class 8 clean room, with a percentage equal to 10% of the final weight and/or volume.

In use, the user can spread and/or inject a varying portion or dose of the product depending on the area to be treated.

According to an application method, the product is first immersed in the autologous blood of the patient himself/herself, for example taken or leaked through the opening point of the surgical site.

The product is then and preferably spread and/or injected and possibly held in place by means of previously arranged adhesives.

The thus-described invention achieves the preset objects. In fact, the use of the reabsorbable device for the reconstruction of cartilage allows the culture to be avoided, which, being rather extensive, is an extremely lengthy, costly and complex operation. It is also possible to avoid regular visits for subsequent intra-articular injections. The reconstruction of cartilage is indeed ensured by the blood impregnating the piece and by the proteins directly from the perforated bone.

The product according to the present invention is also adapted to be spread over porous bone parts, for example for hip revisions, either alone or mixed with other materials used for osseointegration, such as powdered titanium and/or powdered tantalum, as well as to be spread (either alone or possibly mixed with the aforesaid materials) over osteosynthesis means, such as screws or plates to improve the anchoring thereof to the bone parts.

## Claims

1. A reabsorbable product for the reconstruction of cartilage and/or bone parts comprising PGA, said product being obtained by mixing with collagen 1 Kg of PGA granules and/or powder corresponding to the standard ASTM F2313-08 Standard IV 25 (dL/g): 1.10-1.20 Tg: 41 °C Tm: 223°C Max H2O: 0.05% and 0.5kg of hydroxylapatite powder, medical grade, with nominal dimension of 20 micrometres.

2. A product according to claim 1, wherein the granules have a substantially spherical regular shape, and wherein the dimension of the granules ranges from 100 to 900 micrometres, preferably from 200 to 700, even more preferably from 400 to 500.

3. A product according to one of claims 1 and 2, wherein the collagen is present in a percentage such as to give the product a gelatinous consistency.

4. A product according to one of claims 1 to 3, wherein the collagen is collagen of porcine and/or bovine cartilage.

5. A product according to one of claims 1 to 4, wherein the product further comprises supplementary additives such as adhesives and/or amalgamating agents.

6. A product according to claim 5, wherein said supplementary additives comprise biological fibrin.

7. A production method for the production of a product for the regeneration of cartilage and/or bone parts according to one of the claims from 1 to 6, said method comprising grinding of PGA to form said granules and/or powder and mixing with collagen 1kg of PGA corresponding to the standard ASTM F2313-08 Standard IV 25 (dL/g): 1.10-1.20 Tg: 41 °C Tm: 223°C Max H2O: 0.05% and 0.5kg of hydroxylapatite powder, medical grade, with nominal dimension of 20 micrometres.

## Patentansprüche

1. Resorbierbares Produkt zur Rekonstruktion von Knorpelgewebe und/oder Knochenteilen, umfassend PGA, wobei das Produkt erhalten wird durch Mischen mit Kollagen, 1 kg PGA-Granulat und/oder -Pulver, entsprechend dem Standard ASTM F2313-08 Standard IV 25 (dL/g): 1,10-1,20 Tg: 41 °C Tm: 223 °C Max H2O: 0,05 % und 0,5 kg Hydroxylapatit-Pulver medizinischer Güte mit einem Nennmaß von 20 Mikrometer.

2. Produkt nach Anspruch 1, wobei das Granulat eine im Wesentlichen regelmäßige, sphärische Form aufweist und wobei die Abmessung des Granulats im Bereich von 100 bis 900, vorzugsweise von 200 bis 700, mehr bevorzugt von 400 bis 500 Mikrometer liegt.

3. Produkt nach einem der Ansprüche 1 und 2, wobei das Kollagen in einem derartigen Prozentsatz vorhanden ist, dass das Produkt eine gelartige Konsistenz erhält.

4. Produkt nach einem der Ansprüche 1 bis 3, wobei das Kollagen ein Kollagen aus Schweine- und/oder Rinderknorpel ist.

5. Produkt nach einem der Ansprüche 1 bis 4, wobei das Produkt ferner weitere Zusatzstoffe wie Klebstoffe und/oder Mischwirkstoffe umfasst.

6. Produkt nach Anspruch 5, wobei die weiteren Zusatzstoffe biologisches Fibrin umfassen.

7. Herstellungsverfahren zur Herstellung eines Produkts zur Rekonstruktion von Knorpelgewebe und/oder Knochenteilen nach einem der Ansprüche von 1 bis 6, wobei das Verfahren das Mahlen von PGA zur Bildung des Granulats und/oder Pulvers und das Mischen mit Kollagen, 1 kg PGA entsprechend dem Standard ASTM F2313-08 Standard IV 25 (dL/g), umfasst: 1,10-1,20 Tg: 41 °C Tm: 223 °C Max H2O: 0,05 % und 0,5 kg Hydroxylapatit-Pulver medizinischer Güte mit einem Nennmaß von 20 Mikrometer.

## Revendications

1. Produit résorbable pour la reconstruction de cartilage et/ou de parties osseuses comprenant de l'acide folique (PGA), ledit produit étant obtenu par mélange avec du collagène de 1 kg de granules et/ou de poudre de PGA correspondant à la norme ASTM F2313-08 IV 25 (dL/g) : 1,10-1,20 ; Tg : 41°C; Tm : 223°C max ; H2O : 0,05 % et 0,5 kg de poudre d'hydroxyapatite de qualité médicale d'une dimension nominale de 20 micromètres.

2. Produit selon la revendication 1, dans lequel les granules présentent une forme sensiblement sphérique, et dans lequel la dimension des granules est comprise entre 100 et 900 micromètres, de préférence entre 200 et 700, encore plus préférablement entre 400 et 500.

3. Produit selon l'une quelconque des revendications 1 et 2, dans lequel le collagène est présent en un pourcentage tel qu'il permet de donner au produit une consistance gélatineuse.

4. Produit selon l'une quelconque des revendications 1 à 3, dans lequel le collagène est un collagène de cartilage porcin et/ou bovin.

5. Produit selon l'une quelconque des revendications 1 à 4, dans lequel le produit comprend en outre des additifs supplémentaires tels que des adhésifs et/ou des agents amalgamants.

6. Produit selon la revendication 5, dans lequel lesdits additifs supplémentaires comprennent de la fibrine biologique.

7. Procédé de production pour la production d'un produit de régénération de cartilage et/ou de parties osseuses selon l'une quelconque des revendications 1 à 6, ledit procédé comprenant le broyage de PGA pour former lesdites granules et/ou ladite poudre et le mélange avec du collagène de 1 kg de granules et/ou de poudre de PGA correspondant à la norme ASTM F2313-08 IV 25 (dL/g) : 1,10-1,20 ; Tg : 41 °C ; Tm : 223°C max ; H2O : 0,05 % et 0,5 kg de poudre d'hydroxyapatite de qualité médicale d'une dimension nominale de 20 micromètres.
